(19) Europäisches Patentamt
European Patent Office
Office européen des brevets

(11) Veröffentlichungsnummer : **0 440 959 B1**

(12) **EUROPÄISCHE PATENTSCHRIFT**

(45) Veröffentlichungstag der Patentschrift : **16.02.94 Patentblatt 94/07**

(51) Int. Cl.$^5$ : **C07D 285/125**

(21) Anmeldenummer : **90124849.2**

(22) Anmeldetag : **20.12.90**

# (54) Verfahren zur Herstellung von 2-Alkylthio-1,3,4-thiadiazolen.

(30) Priorität : **06.02.90 DE 4003436**

(43) Veröffentlichungstag der Anmeldung : **14.08.91 Patentblatt 91/33**

(45) Bekanntmachung des Hinweises auf die Patenterteilung : **16.02.94 Patentblatt 94/07**

(84) Benannte Vertragsstaaten : **BE CH DE FR GB IT LI NL**

(56) Entgegenhaltungen :
**EP-A- 0 148 501**
**EP-A- 0 165 537**
**EP-A- 0 348 734**
**EP-A- 0 348 736**

(56) Entgegenhaltungen :
**JUSTUS LIEBIGS ANNALEN DER CHEMIE, Band 660, 31. Dezember 1962, Seiten 144-146, Weinheim, DE; H. GEHLEN et al.: "Darstellung von 2-Amino-1.3.4-Oxdiazolen aus acylierten Semicarbaziden"**
**W. THEILHEIMER: "Synthetic methods of organic chemistry", Band 16, 1962, Seite 218, S. Karger, Basel, CH**
**"Methoden der organischen Chemie", Houben-Weyl, Band VIII (1952), S.466-7; Theilheimer, Band 16, 1962, S.227, Nr.482; Chem. Abs., Band 101, 1984, Nr. 90428b**

(73) Patentinhaber : **BAYER AG**
**D-51368 Leverkusen (DE)**

(72) Erfinder : **Diehr, Hans-Joachim, Dr.**
**Höhe 35**
**W-5600 Wuppertal 11 (DE)**

**EP 0 440 959 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues, einstufiges Verfahren zur Herstellung von 2-Methylthio-1,3,4-thiadiazolen, welche als Zwischenprodukte zur Herstellung von Pflanzenschutzmitteln verwendet werden können.

Es ist bekannt, daß man bestimmte 2-Alkylthio-5-halogenalkyl-1,3,4-thiadiazole, wie z.B. 2-Methylthio-5-trifluormethyl-1,3,4-thiadiazol, erhält, wenn man Dithiocarbazinsäure-methylester mit entsprechenden Carbonsäureanhydriden, wie z.B. Trifluoressigsäureanhydrid (vgl. US-P 3 562 284), oder mit entsprechenden Carbonsäuren, wie z.B. Trifluoressigsäure, in einem Lösungsmittel wie Toluol, in Gegenwart von Phosphortrichlorid und Pyridin unter Zusatz von konzentrierter Schwefelsäure (vgl. DE-A-34 22 861) oder mit entsprechenden Carbonsäurechloriden, wie z.B. Trifluoracetylchlorid, in einem Lösungsmittel wie Diethylenglykol-dimethylether, ebenfalls in Gegenwart von Pyridin und unter Zusatz von konzentrierter Schwefelsäure (vgl. DE-A-37 22 320) umsetzt.

Wegen der Verwendung von relativ teuren Carbonsäureanhydriden im Überschuß ist die erstgenannte Synthesemethode aus Kostengründen für die Anwendung im industriellen Maßstab wenig geeignet. Die Umsetzung mit Carbonsäuren, Phosphortrichlorid, Pyridin und Schwefelsäure erfordert ebenso wie die Umsetzung mit Carbonsäurechloriden, Pyridin und Schwefelsäure ein Aufarbeitungsverfahren, bei dem das Pyridin nach der Umsetzung abgetrennt und gegebenenfalls wiedergewonnen wird. Phosphortrichlorid bildet zudem bei der Umsetzung schwerlösliche Produkte, die die Durchmischung erschweren. Bei diesen bekannten Synthesemethoden sind auch die zu erzielenden Ausbeuten nicht ganz zufriedenstellend.

Ferner ist bekannt, daß sich 1-Acyl-semicarbazide durch Kochen mit Phosphorychlorid ($POCl_3$) unter Wasserabspaltung zu den entsprechenden 2-Amino-1,3,4-oxadiazolen cyclisieren lassen; es handelt sich hierbei - insgesamt gesehen - um ein zweistufiges Verfahren, da die 1-Acylgruppen in einer vorgeschalteten Verfahrensstufe in das eigentliche Ausgangsprodukt, Semicarbazid, erst eingeführt werden müssen (vgl. Liebigs Ann. Chem., Band 660, 1962, Seiten 144-146).

Weiterhin vorbeschrieben ist ein zweistufiges Verfahren zur Herstellung von 2-Methylthio-1,3,4-thiadiazolen vom Typ der nachfolgenden Formel (I) durch Umsetzung von geeigneten Carbonsäuren zunächst mit wäßrigen Alkalibasen zu ihren Alkalisalzen, gefolgt von vollständiger Entfernung des Wassers durch azeotrope Destillation, z.B. mittels Toluol, am Wasserabscheider und anschließende Umsetzung der Carbonsäuresalze mit Dithiocarbazinsäuremethylester in Gegenwart von Phosphorychlorid ($POCl_3$) und in Gegenwart eines Verdünnungsmittels, z.B. wieder Toluol (vgl. EP-A-0 148 501, EP-A-0 348 734 und EP-A-0 348 736).

Es wurde nun gefunden, daß man 2-Methylthio-1,3,4-thiadiazole der allgemeinen Formel (I)

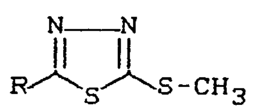

(I),

in welcher

R für einfach bis dreifach durch Fluor und/oder Chlor substituiertes Methyl steht,

mittels einstufiger Verfahrensweise in sehr guten Ausbeuten und in hoher Reinheit erhält, wenn man Carbonsäuren der allgemeinen Formel (II)

$$R\text{-}COOH \qquad (II),$$

in welcher

R die oben angegebene Bedeutung hat,

ausschließlich mit Dithiocarbazinsäure-methylester der Formel (III)

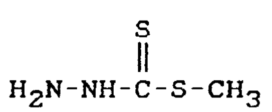

(III),

in Gegenwart von Phosphorylchlorid ($POCl_3$) bei Temperaturen zwischen -20°C und +120°C umsetzt.

Überraschenderweise können nach dem erfindungsgemäßen Verfahren, bei dem anstelle der Kombination Phosphortrichlorid/Pyridin nur Phosphorylchlorid eingesetzt wird, die 2-Methylthio-1,3,4-thiadiazole der

Formel (I) durchweg in besseren Ausbeuten als nach den oben zuerst angegebenen bekannten Verfahren hergestellt werden, wobei weder die Verwendung eines zusätzlichen Lösungs-bzw. Verdünnungsmittels noch die Zugabe von Schwefelsäure erforderlich ist.

Überraschend ist ferner der Befund, daß gemäß dem erfindungsgemäßen Verfahren die freien Carbonsäuren (II) überhaupt reagieren (vgl. Houben-Weyl, Band VIII (1952), Seiten 466-467), so daß die Carbonsäuren nicht zuerst in ihre Salze überführt werden müssen.

Vorteile des erfindungsgemäßen Verfahrens liegen demnach vor allem darin, daß es hinsichtlich der Reaktionskomponenten kostengünstiger und hinsichtlich der Umsetzung und Aufarbeitung unkompliziert ist im Vergleich mit den bekannten Verfahren.

Verwendet man beispielsweise Trifluoressigsäure und Dithiocarbazinsäuremethylester als Ausgangsstoffe, so kann der Reaktionsablauf beim erfindungsgemäßen Verfahren durch das folgende Formelschema skizziert werden:

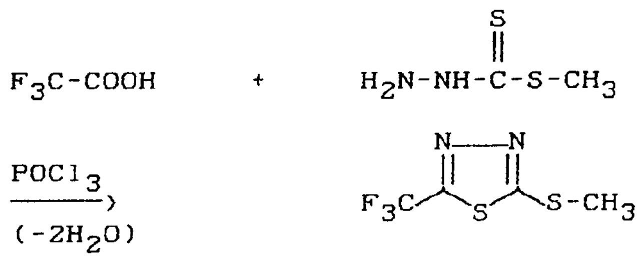


Die beim erfindungsgemäßen Verfahren als Ausgangsstoffe zu verwendenden Carbonsäuren sind durch die Formel (II) allgemein definiert. Als Beispiele für die Ausgangsstoffe der Formel (II) seien genannt: Fluoressigsäure, Difluoressigsäure, Trifluoressigsäure, Chloressigsäure, Dichloressigsäure, Trichloressigsäure, Chlordifluoressigsäure und Fluordichloressigsäure.

Die Ausgangsstoffe der Formel (II) sind bekannte organische Synthesechemikalien.

Dithiocarbazinsäure-methylester der Formel (III) ist bekannt (vgl. z.B. DE-A-19 34 809 und DE-A-37 09 414).

Die Reaktionstemperaturen können bei dem erfindungsgemäßen Verfahren in einem größeren Bereich variiert werden, Im allgemeinen arbeitet man, wie oben angegeben, bei Temperaturen zwischen -20° C und +120° C, vorzugsweise bei Temperaturen zwischen 0° C und +90° C.

Das erfindungsgemäße Verfahren wird im allgemeinen unter Normaldruck durchgefuhrt. Es ist jedoch auch möglich, unter erhöhtem oder vermindertem Druck, im allgemeinen zwischen 0,1 und 10 bar, zu arbeiten.

Zur Durchführung des erfindungsgemäßen Verfahrens setzt man auf 1 Mol Carbonsäure der Formel (II) im allgemeinen zwischen 0,8 und 1,2 Mol, vorzugsweise zwischen 0,95 und 1,05 Mol, Dithiocarbazinsäuremethylester der Formel (III) und zwischen 1 und 10 Mol, vorzugsweise zwischen 1,5 und 5,0 Mol, Phosphorylchlorid ein.

Die Reaktionskomponenten können in beliebiger Reihenfolge zusammengegeben werden.

In einer bevorzugten Ausführungsform des erfindungsgemäßen Verfahrens werden die Carbonsäure der Formel (II) und der Dithiocarbazinsäuremethylester der Formel (III) vorgelegt und das Phosphorylchlorid wird unter Rühren langsam eindosiert.

In einer anderen bevorzugten Variante des erfindungsgemäßen Verfahrens wird zunächst die Carbonsäure der Formel (II) mit dem Phosphorylchlorid vermischt und der Dithiocarbazinsäuremethylester der Formel (III) wird unter Rühren langsam eindosiert,

In beiden Varianten wird dann das Reaktionsgemisch, vorzugsweise bei erhöhter Temperatur, bis zum Ende der Umsetzung gerührt und dann unter vermindertem Druck eingeengt. Der Rückstand wird nach üblichen Methoden aufgearbeitet, Beispielsweise wird mit Eiswasser verrührt, gegebenenfalls angenähert neutralisiert, mit einem mit Wasser praktisch nicht mischbaren organischen Lösungsmittel, wie z.B. Toluol oder Chloroform, geschüttelt, die organische Phase abgetrennt, gegebenenfalls angenähert neutral gewaschen, gegebenenfalls getrocknet und filtriert, Vom Filtrat wird das Lösungsmittel unter vermindertem Druck sorgfältig abdestilliert. Der verbleibende Rückstand enthält im wesentlichen das Produkt der Formel (I), welches im allgemeinen vor der weiteren Verarbeitung nicht weiter gereinigt werden muß.

Die nach dem erfindungsgemäßen Verfahren herzustellenden 2-Methylthio-1,3,4-thiadiazole können als Zwischenprodukte zur Herstellung von Herbiziden oder Mikrobiziden verwendet werden (vgl, US-P 3 562 284, DE-A-3 422 861, DE-A-3 722 320).

Herstellungsbeispiele:

Beispiel 1

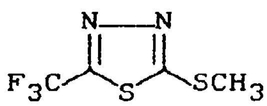

Zu 30,5 g (0,25 Mol) Dithiocarbazinsäure-methylester werden 28,5 g (0,25 Mol) Trifluoressigsäure gegeben. Dann werden zu diesem Gemisch unter Rühren und Kühlen 84 g (0,55 Mol) Phosphorylchlorid tropfenweise gegeben, wobei die Innentemperatur unterhalb von 45° C gehalten wird, Dann wird das Reaktionsgemisch langsam - innerhalb von 3 Stunden - auf eine Innentemperatur von 80° C aufgeheizt (Gasentwicklung!). Nach dem Abklingen der Gasentwicklung wird unter vermindertem Druck eingeengt, der Rückstand mit Eiswasser verrührt, mit 2N-Natronlauge auf pH = 6 eingestellt und das Produkt mit 150 ml Toluol extrahiert. Die toluolische Lösung wird mit 5%iger Natriumhydrogencarbonat-Lösung und dann mit Wasser gewaschen; dann wird das Lösungsmittel im Wasserstrahlvakuum bei 50° C (Badtemperatur) sorgfältig abdestilliert.

Man erhält 62 g eines gelben Öles, das nach gaschromatographischer Analyse 75% 2-Methylthio-5-trifluormethyl-1,3,4-thiadiazol enthält (Rest: Toluol); Ausbeute: 93% der Theorie.

Analog Beispiel 1 und gemäß der allgemeinen Beschreibung des erfindungsgemäßen Verfahrens können beispielsweise auch die in der nachstehenden Tabelle 1 aufgeführten Verbindungen der Formel (I) hergestellt werden,

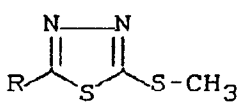

(I)

Tabelle 1:

| Bsp.-Nr. | R | Siedepunkt/Druck (°C/mbar) (oder: Schmelzpunkt Fp.) | Ausbeute (% d.Th.) |
|---|---|---|---|
| 2 | $ClF_2C$- | 120/20 | 89 |
| 3 | $FCl_2C$- | 96-97/2 | |
| 4 | $Cl_3C$- | (Fp.: 31° C) | |
| 5 | $F_2CH$- | 73-74/1 | |
| 6 | $Cl_2CH$- | 112/1 | |
| 7 | $ClCH_2$- | (Fp.: 68° C) | |

Die Verbindung gemäß Beispiel 1 kann erfindungsgemäß beispielsweise auch wie folgt hergestellt werden:

Beispiel 1a

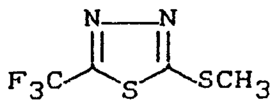

In ein Gemisch aus 100 ml Phosphorylchlorid und 57 g (0,5 Mol) Trifluoressigsäure werden unter Rühren und Eiskühlung portionsweise - innerhalb einer halben Stunde - 61 g (0,5 Mol) Dithiocarbazinsäure-methylester eingetragen. Das Reaktionsgemisch wird anschließend 15 Minuten ohne Kühlung gerührt, dann langsam auf 80° C aufgeheizt und bei dieser Temperatur bis zum Abklingen der Gasentwicklung gerührt. Nach Einengen unter vermindertem Druck wird der Rückstand mit Toluol/Eiswasser verrührt; die organische Phase wird anschließend abgetrennt, mit Wasser gewaschen und unter vermindertem Druck eingeengt.
Man erhält 96,5 g eines gelben Öles, das nach gaschromatografischer Analyse 98,5% 2-Methylthio-5-trifluormethyl-1,3,4-thiadiazol enthält;
Ausbeute: 95% der Theorie.

**Patentansprüche**

1. Einstufiges Verfahren zur Herstellung von 2-Methylthio-1,3,4-thiadiazolen der Formel (I)

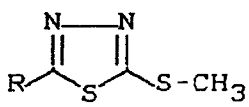

(I),

in welcher
R für einfach bis dreifach durch Fluor und/oder Chlor substituiertes Methyl steht,
dadurch gekennzeichnet, daß man Carbonsäuren der Formel (II)

R-COOH (II),

in welcher
R die oben angegebene Bedeutung hat,
ausschließlich mit Dithiocarbazinsäure-methylester der Formel (III)

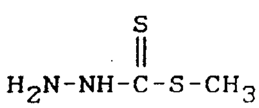

(III)

in Gegenwart von Phosphorylchlorid ($POCl_3$) bei Temperaturen zwischen -20° C und +120° C umsetzt.

2. Verfahren nach Anspruch 1, dadurch gekennzeichnet, daß man bei Temperaturen zwischen 0° und +90° C arbeitet.

3. Verfahren nach Anspruch 1, dadurch gekennzeichnet, daß man auf 1 Mol Carbonsäure (II) 0,8 bis 1,2 Mol, vorzugsweise 0,95 bis 1,05 Mol, Dithiocarbazinsäure-methylester (III) einsetzt.

4. Verfahren nach Anspruch 1, dadurch gekennzeichnet, daß man auf 1 Mol Carbonsäure (II) 1 bis 10 Mol, vorzugsweise 1,5 bis 5 Mol, Phosphorylchlorid ($POCl_3$) einsetzt.

5. Verfahren nach Anspruch 1, dadurch gekennzeichnet, daß man die Carbonsäure (II) und den Dithiocarbazinsäure-methylester (III) vorlegt und das Phosphorylchlorid unter Rühren langsam eindosiert.

6. Verfahren nach Anspruch 1, dadurch gekennzeichnet, daß man zunächst die Carbonsäure (II) mit dem Phosphorylchlorid vermischt und dann den Dithiocarbazinsäure-methylester (III) unter Rühren langsam eindosiert.

## Claims

1. One-step process for the preparation of 2-methylthio-1,3,4-thiadiazoles of the formula (I)

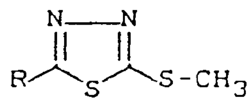

(I),

in which
R represents methyl which is monosubstituted to trisubstituted by fluorine and/or chlorine,
characterized in that carboxylic acids of the formula (II)

R-COOH (II),

in which
R has the abovementioned meaning,
are reacted exclusively with methyl dithiocarbazate, of the formula (III)

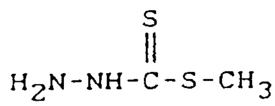

(III),

in the presence of phosphoryl chloride ($POCl_3$) at temperatures between -20°C and +120°C.

2. Process according to Claim 1, characterized in that the process is carried out at temperatures between 0° and +90°C.

3. Process according to Claim 1, characterized in that 0.8 to 1.2 moles, preferably 0.95 to 1.05 moles, of methyl dithiocarbazate (III) are employed per mole of carboxylic acid (II).

4. Process according to Claim 1, characterized in that 1 to 10 moles, preferably 1.5 to 5 moles, of phosphoryl chloride ($POCl_3$) are employed per mole of carboxylic acid (II).

5. Process according to Claim 1, characterized in that the carboxylic acid (II) and the methyl dithiocarbazate (III) are initially introduced, and the phosphoryl chloride is slowly metered in with stirring.

6. Process according to Claim 1, characterized in that the carboxylic acid (II) is first mixed with the phosphoryl chloride, and the methyl dithiocarbazate (III) is then slowly metered in with stirring.

## Revendications

1. Procédé en une étape pour la préparation de 2-méthylthio-1,3,4-diathiazoles de formule (I)

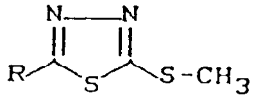

(I),

dans laquelle

R représente un radical méthyle substitué de une à trois fois par le fluor et/ou le chlore,

caractérisé en ce que l'on fait réagir des acides carboxyliques de formule (II)

R-COOH (II)

dans laquelle

R a la signification précitée exclusivement

avec l'ester méthylique de l'acide dithiocarbazinique de formule (III)

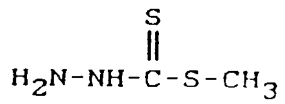

(III),

en présence de chlorure de phosphoryle ($POCl_3$) à des températures comprises entre -20°C et +120°C.

2. Procédé selon la revendication 1,caractérisé en ce que l'on opère à des températures entre 0 et +90°C.

3. Procédé selon la revendication 1,caractérisé en ce que l'on utilise pour une mole d'acide carboxylique (II) de 0,8 à 1,2 mole et, de préférence, de 0,95 à 1,05 mole d'ester méthylique de l'acide dithiocarbazinique (III).

4. Procédé selon la revendication 1,caractérisé en ce que l'on utilise pour une mole d'acide carboxylique (II) de 1 à 10 moles et, de préférence, de 1,5 à 5 moles de chlorure de phosphoryle ($POCl_3$).

5. Procédé selon la revendication 1,caractérisé en ce que l'on ajoute tout d'abord l'acide carboxylique (II) et l'ester méthylique de l'acide dithiocarbazinique (III) et que l'on ajoute ensuite lentement avec agitation le chlorure de phosphoryle.

6. Procédé selon la revendication 1,caractérisé en ce que l'on mélange tout d'abord l'acide carboxylique (II) au chlorure de phosphoryle et que l'on ajoute ensuite lentement, avec agitation, l'ester méthylique de l'acide dithiocarbazinique (III).